# FASCICULE DE BREVET EUROPEEN

(11) **EP 1 996 272 B1**
(45) Date de publication et mention de la délivrance du brevet: **27.01.2010**
(21) Numéro de dépôt: 07731139.7
(22) Date de dépôt: 15.03.2007
(51) Int. Cl.: A61M 25/01, A61M 39/12

(54) **INTRODUCTEUR DE CATHETER PAR VOIE OMBILICALE ET KIT**
VORRICHTUNG ZUR EINFÜHRUNG EINES KATHETERS AUF DEM UMBILIKALEN WEG UND KIT
DEVICE FOR INTRODUCING A CATHETER BY THE UMBILICAL ROUTE, AND KIT

(30) Priorité: 16.03.2006 FR 0602300
(43) Date de publication de la demande: 03.12.2008
(73) Titulaire: VYGON, F-95440 Ecouen (FR)
(72) Inventeur: HAUMONT, Dominique, 1180 Bruxelles (BE); LETANG, Fabien, 60410 Verberie (FR)
(74) Mandataire: Le Forestier, Eric
(86) Numéro de dépôt international: PCT/FR2007/000445
(87) Numéro de publication internationale: WO 2007/104866

(56) Documents cités:
- WO-A-81/01519
- WO-A-96/33764
- US-A- 5 789 018
- US-A1- 2005 209 572

## Description

L'invention concerne un introducteur de cathéter veineux central par voie ombilicale chez les bébés prématurés ou nouveau-nés.

On a proposé pour cette application d'utiliser un cathéter en silicone introduit au moyen d'un cathéter ombilical en polyuréthane (Amato M.Weissmüller A, Hüppi P : « Umbilicous venous silastic cathéter- a new procedure in low-birth-weight infants » Neonatologica 1992 (2), 117-20, et Scharrer B, Rudin C, Nars PW : « Transumbilical venous access with small diameter silastic cathéters in very low birth weight infants » Eur J Pediatr. 1997, 156(11) : 897-8.)

Les cathéters en silicone sont mieux tolérés par les organismes que ceux en polyuréthane. Par contre, ils sont très mous et de ce fait difficiles à introduire. Par la voie ombilicale, on touche les grosses veines des nouveau-nés, qui sont en dépression, et le risque d'embolie gazeuse par entrée d'air est important surtout en cas d'inspiration forte. L'embolie gazeuse est mortelle.

Les cathéters en polyuréthane sont relativement moins souples que les cathéters silicone mais il est difficile d'assurer la progression d'un cathéter en silicone dans un cathéter en polyuréthane si les deux cathéters sont suffisamment ajustés l'un à l'autre pour éviter une entrée d'air entre les cathéters.

Le document WO 96/33769 A décrit un système pour introduire un cathéter dans une veine comprenant un canule elle-même comprenant une gaine tubulaire allongée comprise entre une fin distale et une fin proximale de la canule, et un embout entourant la fin proximale de la gaine. La canule est adaptée pour recevoir une aiguille d'introduction permettant d'introduire la fin distale de la gaine tubulaire de la canule dans une veine.

Le document US-A-5 789 018 décrit des cathéters pouvant être utilisés lors de procédures cardiovasculaires et endovasculaires pour délivrer des agents thérapeutiques, diagnostiques ou vaso-occlusifs à une région ciblée d'un corps humain ou animal accessibles par voies naturelles. Les cathéters sont enduits de telle manière qu'il sont glissants et que l'enduit est durable. Le document décrit aussi des méthodes de dépôt de l'enduit et des méthodes de dépôt de lubrifiant par formation d'une couche d'enduit sur substrat accompagné d'un séchage simultané et de création de liaisons transversales par de la chaleur ou par des radiations.

Le document WO 81/01519 A décrit un cathéter destiné à l'usage sur des nouveau-nés comprenant un embout muni d'une chambre, d'un tube, une première partie du tube est translucide et une deuxième partie est radio-opaque. La première partie du tube est connectée à l'embout par un insert et la fin proximale de la deuxième partie du tube s'étend par engagement hermétique par dessus la fin distale de la première section pour former une connexion flexible. Le cathéter incluant préférentiellement un stylet facilitant l'insertion.

Le document US 2005/209572 A1 décrit un cathéter muni d'une valve permettant un placement et enlèvement d'un appareil ou d'un élément attaché sans risque d'embolie par air ou de perte de sang. Le cathéter avec valve inclus un tube ayant à sa fin proximale un col formant une valve intégrale, le col est entouré par une gaine de compression qui biaise la valve dans une position fermée.

La présente invention a pour objet un introducteur de cathéter veineux central en silicone,par voie ombilicale, chez les bébés prématurés ou nouveau-nés, comportant un cathéter en polyuréthane et facilitant le glissement du cathéter en silicone dans le cathéter en polyuréthane tout en éliminant les risques d'embolie gazeuse.

Selon l'invention, l'introducteur, qui comprend un cathéter en polyuréthane dans lequel peut glisser le cathéter en silicone, comprend également un tronçon de tube en métal inoxydable, rectiligne, rigide, plus court que le cathéter en polyuréthane et dont le diamètre intérieur est ajusté au diamètre extérieur du cathéter en silicone pour permettre un glissement du cathéter en silicone dans le tube métallique tout en empêchant une entrée d'air entre eux, ce tronçon de tube métallique étant introduit dans l'extrémité proximale du cathéter en polyuréthane et fixé à ce cathéter de façon à empêcher une entrée d'air entre eux.

Le tube en métal inoxydable est idéal, car il permet la précision nécessaire (+/- 0, 01mm) et offre une glisse correcte au contact du cathéter en silicone.

De part sa rigidité, le tube métallique permet d'introduire le cathéter en silicone sans que celui-ci se bloque. Il constitue un mandrin externe. On peut pousser sur le cathéter en silicone qui ne peut que rester droit. Il reste au cathéter en silicone à parcourir une longueur dans le cathéter en polyuréthane d'environ 3 à 6 cm avant d'arriver dans la veine et d'être « aspiré » par la légère dépression (retour du sang au coeur).

Pour l'introduction d'un cathéter en silicone de diamètre interne 0, 3mm et de diamètre externe 0, 6 mm, long de 15 à 50 cm, l'introducteur de l'invention est constitué de :
- un tube en métal inoxydable de φ 0, 8 x φ 1, 0 mm et de longueur environ 45 mm,
- un cathéter en polyuréthane de 5 Fr de φ 1, 0 x 1, 7 mm et de longueur 5 à 10 cm.

De préférence, le tube métallique est équipé d'une embase souple à deux ailettes pour la prise en main de l'introducteur. Il est introduit par serrage et collage dans le cathéter en polyuréthane (qui présente en plus plusieurs lignes radio opaques détectables aux rayons X). Le cathéter en polyuréthane a la longueur suffisante pour entrer à l'endroit voulu dans la veine ombilicale. Selon la taille des bébés, deux longueurs sont prévues : 6 à 9 cm.

L'extrémité distale du cathéter en polyuréthane peut être émoussée éventuellement pour éviter toute forme agressive, et ce cathéter peut être marqué par centimètres pour repérer la longueur insérée dans la veine.

Le tube métallique permet la poussée et la non-entrée d'air de par sa rigidité, sa glisse, ses tolérances précises.

L'introducteur permet de pousser le cathéter très fin et très mou en silicone sans risque de coincement, il permet de pousser le cathéter en silicone et de retirer l'introducteur sans risque d'embolie gazeuse.

Sur les figures du dessin joint :

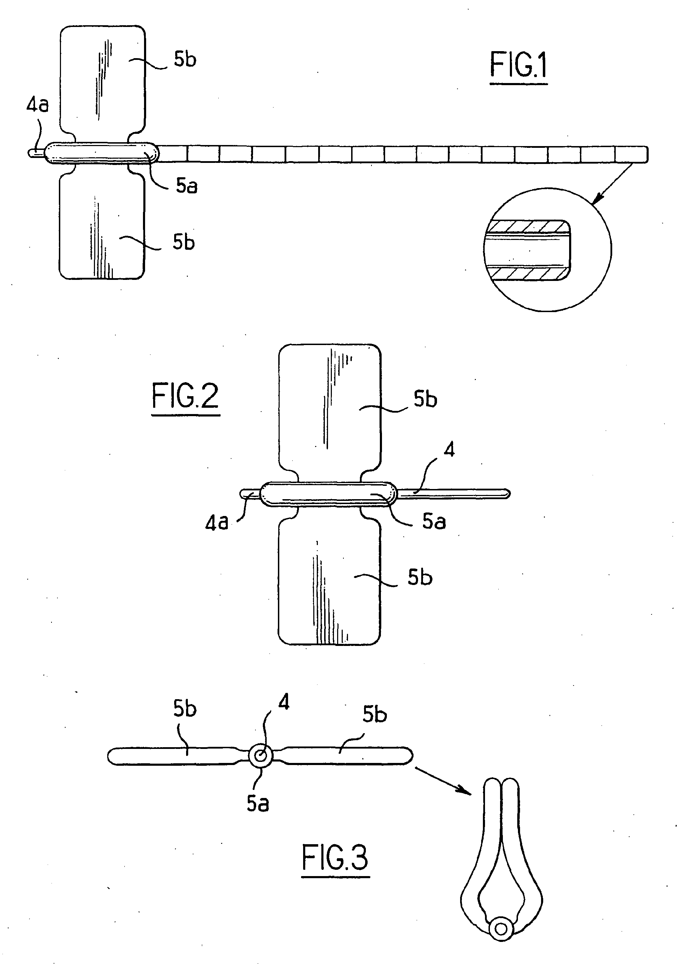

- la figure 1 est une vue schématique en plan d'un introducteur selon l'invention ;
- la figure 2 est un schéma en plan du tube métallique ;
- la figure 3 est une coupe transversale du tube métallique au niveau des ailettes de préhension ;

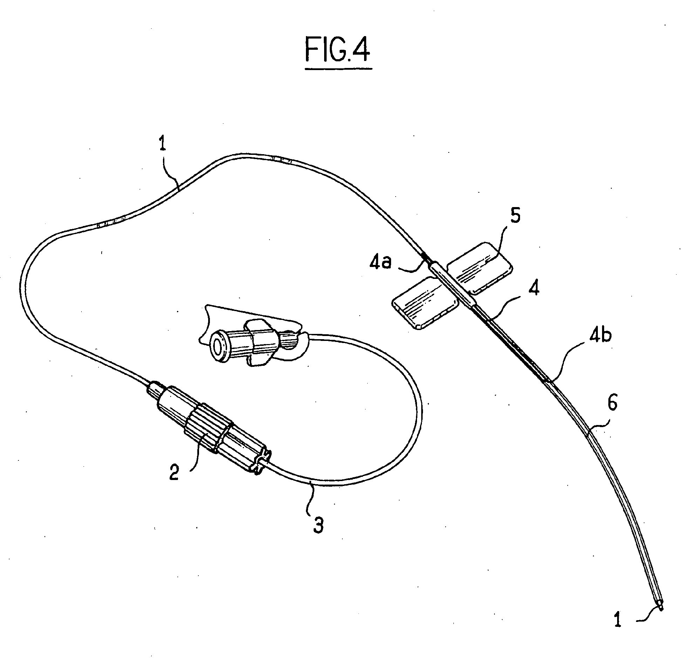

- la figure 4 est une vue d'un kit constitué de l'introducteur de la figure 1 et d'un cathéter en silicone, et
- les figures 5, 6 et 7 sont des schémas de mise en oeuvre.

L'introducteur comprend un tube rectiligne en métal inoxydable (4) relativement court qui présente une extrémité proximale (4a) équipée d'une embase de préhension (5) constituée d'un fût (5a) et de deux ailettes (5b).

Le tube métallique est introduit dans une extrémité d'un cathéter souple en polyuréthane (6) qui est sensiblement plus long que le tube métallique. Le tube et le cathéter sont fixés par collage.

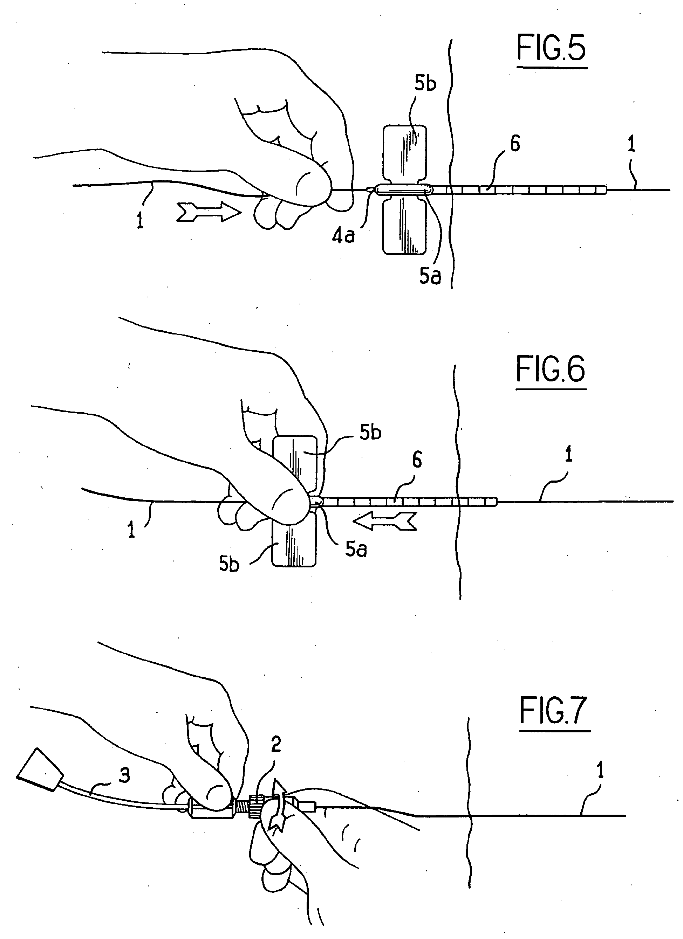

Pour sa mise en place, le cathéter en silicone est glissé dans le tube métallique puis dans le cathéter en polyuréthane (figure 5). Pour retirer le tube métallique et le tube en polyuréthane, cet ensemble est tenu par les ailettes et coulissé vers l'arrière sur le cathéter en silicone (figure 6).

Le cathéter en silicone (1) après retrait du cathéter en polyuréthane peut être équipé d'une embase proximale amovible (2) permettant son raccordement à un dispositif (3) (figure 7).

L'invention n'est pas limitée à cet exemple de réalisation. En particulier les matériaux cités pourraient être remplacés par des matériaux fonctionnellement équivalents ou ayant des qualités supérieures.

L'invention n'est pas limitée à la réalisation qui a été décrite.

## Revendications

1. Introducteur de cathéter en silicone (1), par voie ombilicale, qui comprend un cathéter souple (6) en polyuréthane dans lequel peut glisser le cathéter en silicone, **caractérisé en ce qu'**il comprend également un tronçon de tube (4) en métal inoxydable, rectiligne, rigide, plus court que le cathéter en polyuréthane et dont le diamètre intérieur est ajusté au diamètre extérieur du cathéter en silicone pour permettre un glissement du cathéter en silicone dans le tube métallique tout en empêchant une entrée d'air entre eux, ce tube métallique étant introduit dans l'extrémité proximale du cathéter en polyuréthane et fixé à ce cathéter de façon à empêcher une entrée d'air entre eux.

2. Introducteur selon la revendication 1 dont le tube en métal inoxydable (4) est pourvu d'une embase proximale à ailettes (5).

3. Introducteur selon la revendication 1 ou 2 dont le cathéter en polyuréthane (6) présente des lignes radio opaques détectables aux rayons X.

4. Kit comprenant un introducteur selon l'une des revendications 1 à 3 et un cathéter en silicone (1), le diamètre intérieur du tube (4) en métal inoxydable étant ajusté au diamètre extérieur du cathéter silicone pour permettre le coulissement du cathéter tout en évitant une entrée d'air entre eux.

5. Kit selon la revendication 4 dans lequel le cathéter en silicone (1) est équipé d'une embase proximale amovible (2).

6. Kit selon la revendication 4 ou 5 et qui comprend :
• un cathéter en silicone de 2 Fr (0, 3 x 0, 6 mm) long de 15 à 50 cm,
• un tube en métal inoxydable de φ 0, 8 x φ 1, 0 mm et de longueur 45 mm environ,
• un cathéter en polyuréthane de 5 Fr de φ 1, 0 x 1, 7 mm et de longueur 5 à 10 cm.

## Claims

1. Device for introducing a silicone catheter (1), by the umbilical route, which comprises a flexible polyurethane catheter (6) in which the silicone catheter can slide, **characterised in that** it also comprises a section of rectilinear and rigid tube (4), which is made of stainless metal and is shorter than the catheter and whose internal diameter is adapted to the external diameter of the silicone catheter in order to allow the silicone catheter to slide in the metal tube, while preventing entry of air between them, this metal tube being introduced into the proximal end of the polyurethane catheter and fixed to said catheter in such a way as to prevent entry of air between them.

2. Introduction device according to claim 1 wherein the stainless metal tube (4) is provided with a proximal base with fins (5).

3. Introduction device according to claim 1 or 2 wherein the polyurethane catheter (6) displays X-ray detectable opaque radio lines.

4. Kit comprising an introduction device according to any one of claims 1 to 3 and a silicone catheter (1), the internal diameter of the stainless metal tube (4) being adapted to the external diameter of the silicone catheter to enable the sliding of the catheter while preventing entry of air between them.

5. Kit according to claim 4 wherein the silicone catheter (1) is equipped with a removable proximal base (2).

6. Kit according to claim 4 or 5 comprising:
- a 2 Fr silicone catheter (0.3 x 0.6 mm) 15 to 50 cm in length,
- a stainless metal tube 0.8 x 1.0 mm in diameter and approximately 45 mm in length,
- a 5 Fr polyurethane catheter 1.0 x 1.7 mm in diameter and 5 to 10 cm in length.

## Patentansprüche

1. Einführungsvorrichtung für Katheter aus Silikon (1) über den Nabel, die einen biegsamen Katheter (6) aus Polyurethan umfasst, in dem der Katheter aus Silikon gleiten kann, **dadurch gekennzeichnet, dass** sie ebenfalls einen geradlinigen und steifen Röhrenabschnitt (4) aus rostfreiem Metall umfasst, der kürzer ist als der Katheter aus Polyurethan und dessen Innendurchmesser auf den Außendurchmesser des Katheters aus Silikon abgestimmt ist, um ein Gleiten des Katheters aus Silikon in der metallischen Röhre zu gestatten und dabei gleichzeitig einen Lufteintritt zwischen ihnen zu verhindern, wobei die metallische Röhre in das proximale Ende des Katheters aus Polyurethan eingeführt ist und an diesem Katheter derart befestigt ist, dass ein Lufteintritt zwischen ihnen verhindert wird.

2. Einführungsvorrichtung nach Anspruch 1, deren Röhre aus rostfreiem Metall (4) mit einem proximalen Basisteil mit Flügeln (5) ausgestattet ist.

3. Einführungsvorrichtung nach Anspruch 1 oder 2, deren Katheter aus Polyurethan (6) strahlungsopake Linien aufweist, die mit Röntgenstrahlen detektierbar sind.

4. Set, der eine Einführungsvorrichtung nach einem der Ansprüche 1 bis 3 und einen Katheter aus Silikon (1) umfasst, wobei der Innendurchmesser der Röhre (4) aus rostfreiem Metall auf den Außendurchmesser des Silikonkatheters abgestimmt ist, um das Gleiten des Katheters zu.gestatten und dabei gleichzeitig einen Lufteintritt zwischen ihnen zu verhindern.

5. Set nach Anspruch 4, bei dem der Katheter aus Silikon (1) mit einem abnehmbaren proximalen Anschlussteil (2) ausgestattet ist.

6. Set nach Anspruch 4 oder 5, außerdem folgendes umfassend:
• einen Katheter aus Silikon von 2 Fr (0,3 x 0,6 mm) mit einer Länge von 15 bis 50 cm,
• eine Röhre aus rostfreiem Metall mit φ 0,8 x φ 1,0 mm und einer Länge von etwa 45 mm,
• einen Katheter aus Polyurethan von 5 Fr mit φ 1,0 x 1,7 mm und einer Länge von 5 bis 10 cm.
